# EUROPEAN PATENT SPECIFICATION

(11) **EP 3 914 247 B1**
(45) Date of publication and mention of the grant of the patent: **13.11.2024**
(21) Application number: 20708283.5
(22) Date of filing: 22.01.2020
(51) Int. Cl.: A61K 31/445, A61P 9/00, A61P 25/00

(54) **USE OF MIGALOSTAT IN REDUCING THE RISK OF CEREBROVASCULAR EVENT IN PATIENTS WITH FABRY DISEASE**
VERWENDUNG VON MIGALOSTAT ZUR VERRINGERUNG DES RISIKOS DES ZEREBROVASKULÄREN EREIGNISSES BEI PATIENTEN MIT FABRY-KRANKHEIT
UTILISATION DE MIGALOSTAT POUR RÉDUIRE LE RISQUE D'ACCIDENT VASCULAIRE CÉRÉBRAL CHEZ LES PATIENTS SOUFFRANT DE LA MALADIE DE FABRY

(30) Priority: 22.01.2019 US 201962795524 P
(43) Date of publication of application: 01.12.2021
(73) Proprietor: Amicus Therapeutics, Inc., Philadelphia, PA 19104 (US)
(72) Inventor: SKUBAN, Nina, Cranbury, New Jersey 08512 (US); LAGAST, Hjalmar, Cranbury, New Jersey 08512 (US)
(74) Representative: Hoffmann Eitle
(86) International application number: PCT/US2020/014531
(87) International publication number: WO 2020/154354

(56) References cited:
- GERE SUNDER-PLASSMANN ET AL: "Migalastat for the treatment of Fabry disease", EXPERT OPINION ON ORPHAN DRUGS, vol. 6, no. 5, 4 May 2018 (2018-05-04), pages 301 - 309, XP055527565, DOI: 10.1080/21678707.2018.1469978
- DERRALYNN A HUGHES ET AL: "Oral pharmacological chaperone migalastat compared with enzyme replacement therapy in Fabry disease: 18-month results from the randomised phase III ATTRACT study", JOURNAL OF MEDICAL GENETICS, vol. 54, no. 4, 10 November 2016 (2016-11-10), GB, pages 288 - 296, XP055690446, ISSN: 0022-2593, DOI: 10.1136/jmedgenet-2016-104178
- RANIERI MICHELA ET AL: "Fabry Disease: Recognition, Diagnosis, and Treatment of Neurological Features", CURRENT TREATMENT OPTIONS IN NEUROLOGY, SPRINGER US, BOSTON, vol. 18, no. 7, 26 May 2016 (2016-05-26), pages 1 - 18, XP035989484, ISSN: 1092-8480, [retrieved on 20160526], DOI: 10.1007/S11940-016-0414-5
- SHENG SEN ET AL: "Fabry's disease and stroke: Effectiveness of enzyme replacement therapy (ERT) in stroke prevention, a review with meta-analysis", JOURNAL OF CLINICAL NEUROSCIENCE, vol. 65, 4 April 2019 (2019-04-04), pages 83 - 86, XP085707717, ISSN: 0967-5868, DOI: 10.1016/J.JOCN.2019.03.064

## Description

### TECHNICAL FIELD

Principles and embodiments of the present invention relate generally to the use of pharmacological chaperones for the treatment of lysosomal storage disorders, particularly the use of migalastat for the treatment of Fabry disease.

### BACKGROUND

Fabry disease is a progressive, X-linked inborn error of glycosphingolipid metabolism caused by a deficiency in the lysosomal enzyme α-galactosidase A (α-Gal A) as a result of mutations in the α-Gal A gene (GLA). Despite being an X-linked disorder, females can express varying degrees of clinical manifestations. Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

One approved therapy for treating Fabry disease is enzyme replacement therapy (ERT), which typically involves intravenous, infusion of a purified form of the corresponding wild-type protein. Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). While ERT is effective in many settings, the treatment also has limitations. ERT has not been demonstrated to decrease the risk of stroke, cardiac muscle responds slowly, and GL-3 elimination from some of the cell types of the kidneys is limited. Some patients also develop immune reactions to ERT.

Accordingly, there remains a need for therapies for the treatment of Fabry disease, especially for reducing the risk of cerebrovascular events.

Sunder-Plassmann et al. (Expert Opinion on Orphan Drugs, 6:5, 301-309; 2018) generally describe migalastat for the treatment of Fabry disease. Hughes et al. (J Med Genet, 54(4): 288-296; 2017) describe the oral pharmacological chaperone migalastat compared with enzyme replacement therapy in Fabry disease, and in particular 18-month results from the randomised phase III ATTRACT study. Ranieri et al. (Curr Treat Options Neurol, 18(7): 33; 2016) describe the recognition, diagnosis, and treatment of neurological features of Fabry disease.

### SUMMARY

The present invention is defined by the appended claims. Various aspects of the present invention relate to migalastat or salt thereof for use in the treatment of Fabry disease in ERT-naive and ERT-experienced patients Such treatment reduces the risk of cerebrovascular (CBV) events. The treatment includes long-term migalastat therapy, such as treatment for at least 2, 3, 4 or more years.

One aspect of the present invention pertains to migalastat or salt thereof for use in a method of reducing the risk of a CBV event in a patient having Fabry disease, the method comprising administering to the patient a formulation comprising an effective amount of migalastat or salt thereof every other day, for at least 2 years, wherein the effective amount is about 100 mg to about 150 mg free base equivalent (FBE).

In one or more embodiments, the CBV event comprises one or more of brain stem ischemia, cerebral infarction, cerebral hemorrhage, cerebral ischemia, cerebrovascular accident, embolic stroke or transient ischemic attack.

In one or more embodiments, the patient has an increased risk of a CBV event prior to initiating administration of the migalastat or salt thereof.

In one or more embodiments, the migalastat or salt thereof enhances α-Gal A activity.

In one or more embodiments, the patient is administered about 123 mg FBE of the migalastat or salt thereof every other day.

In one or more embodiments, the patient is administered about 123 mg of migalastat free base every other day.

In one or more embodiments, the patient is administered about 150 mg of migalastat hydrochloride every other day.

In one or more embodiments, the formulation comprises an oral dosage form. In one or more embodiments, the oral dosage form comprises a tablet, a capsule or a solution.

The migalastat or salt thereof is administered for at least 2 years.

In one or more embodiments, the migalastat or salt thereof is administered for at least 3 years.

In one or more embodiments, the migalastat or salt thereof is administered for at least 4 years.

In one or more embodiments, the patient is an ERT-naive patient.

In one or more embodiments, the patient is an ERT-experienced patient.

In one or more embodiments, the patient has a HEK assay amenable mutation in α-galactosidase A. In one or more embodiments, the mutation is disclosed in a pharmacological reference table. In one or more embodiments, the pharmacological reference table is provided in a product label for a migalastat product approved for the treatment of Fabry disease. In one or more embodiments, the pharmacological reference table is provided in a product label for GALAFOLD^{®}. In one or more embodiments, the pharmacological reference table is provided at a website. In one or more embodiments, the website is one or more of www.galafoldamenabilitytable.com or www.fabrygenevariantsearch.com.

### BRIEF DESCRIPTION OF THE DRAWINGS

Further features of the present invention will become apparent from the following written description and the accompanying figures, in which:
FIGS. 1A-E show the full DNA sequence of the human wild-type GLA gene (SEQ ID NO: 1);
FIG. 2 shows the wild-type α-Gal A protein (SEQ ID NO: 2); and
FIG. 3 shows the nucleic acid sequence encoding the wild-type α-Gal A protein (SEQ ID NO: 3).

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Various aspects of the present invention pertain to dosing regimens for the administration of migalastat for the treatment of Fabry disease. The dosing regimens of migalastat reduce the risk of a CBV event.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb₃").

The term "atypical Fabry disease" refers to patients with primarily cardiac manifestations of the α-Gal A deficiency, namely progressive GL-3 accumulation in myocardial cells that leads to significant enlargement of the heart, particularly the left ventricle.

A "carrier" is a female who has one X chromosome with a defective α-Gal A gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

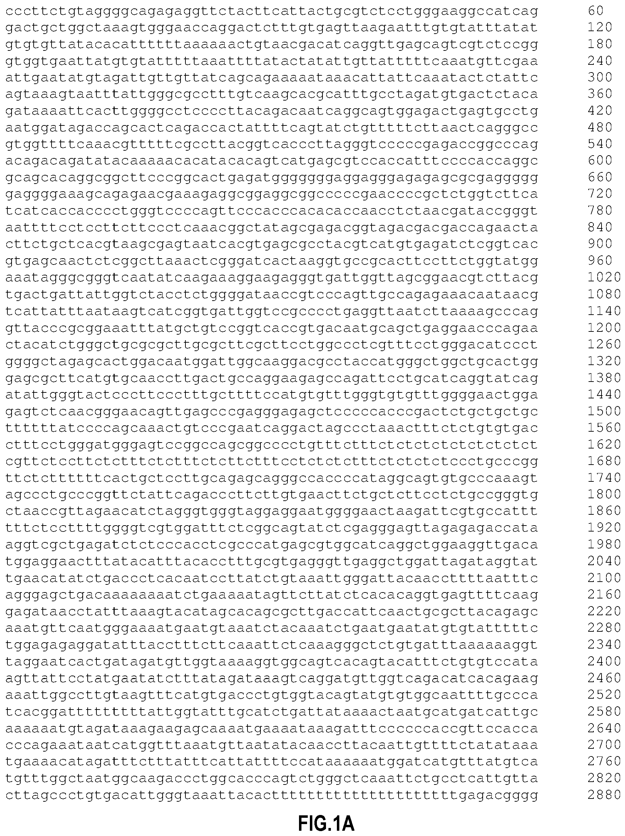

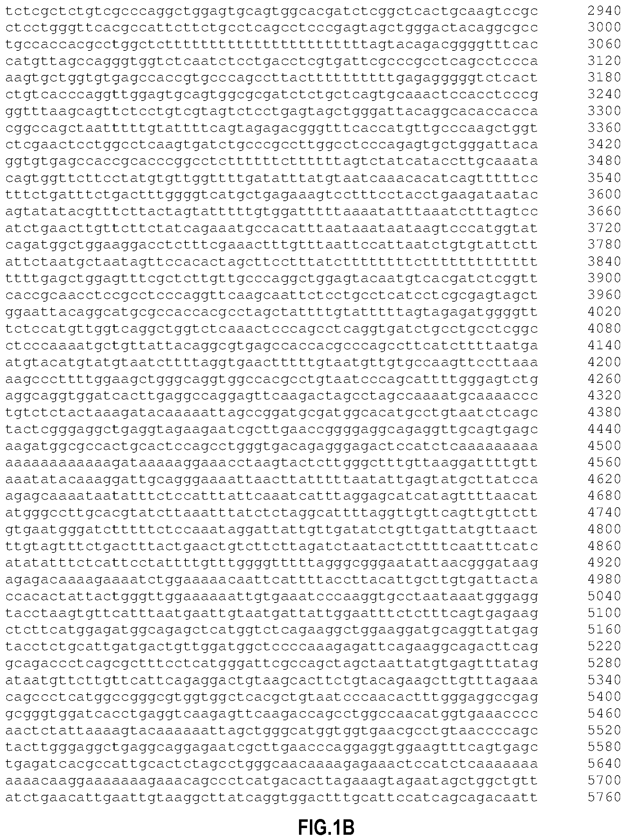

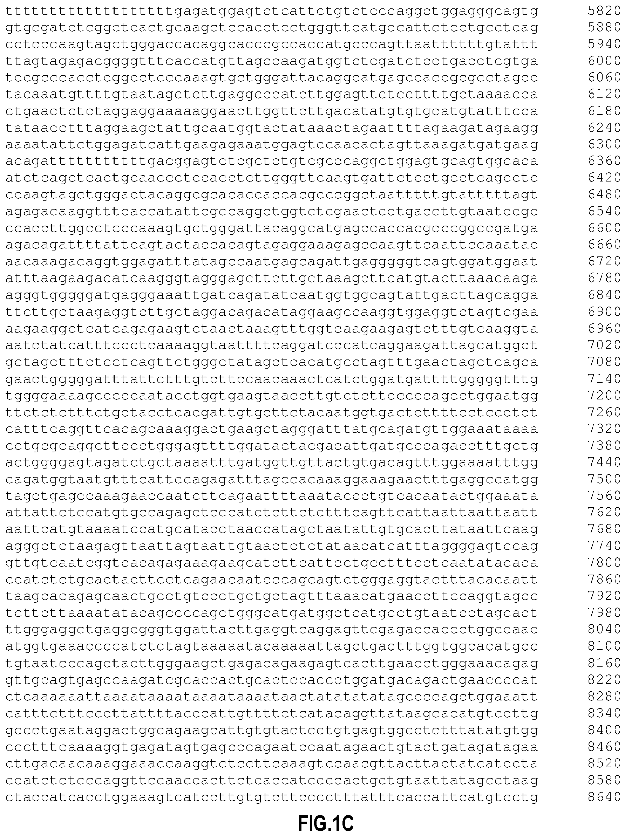

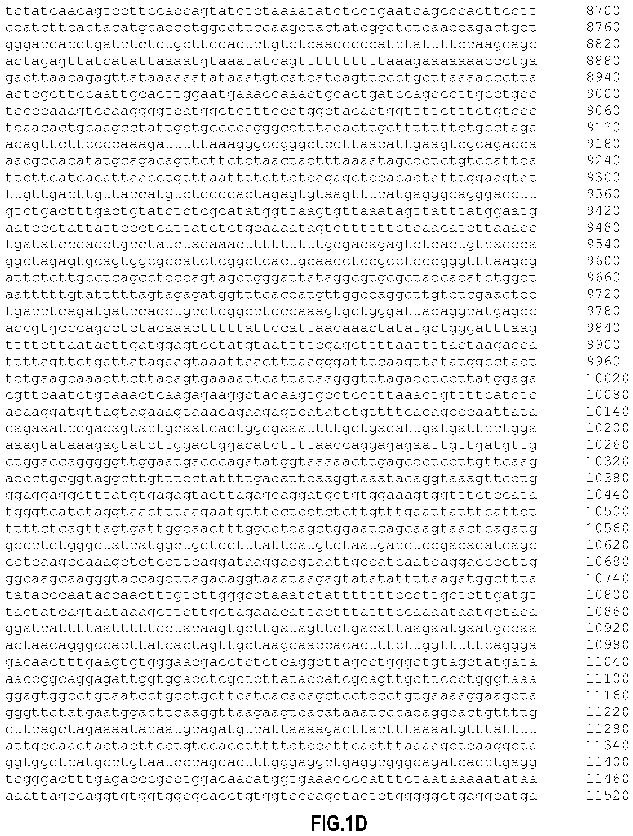

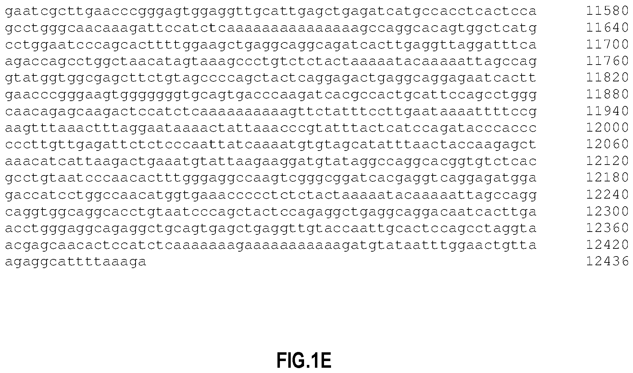

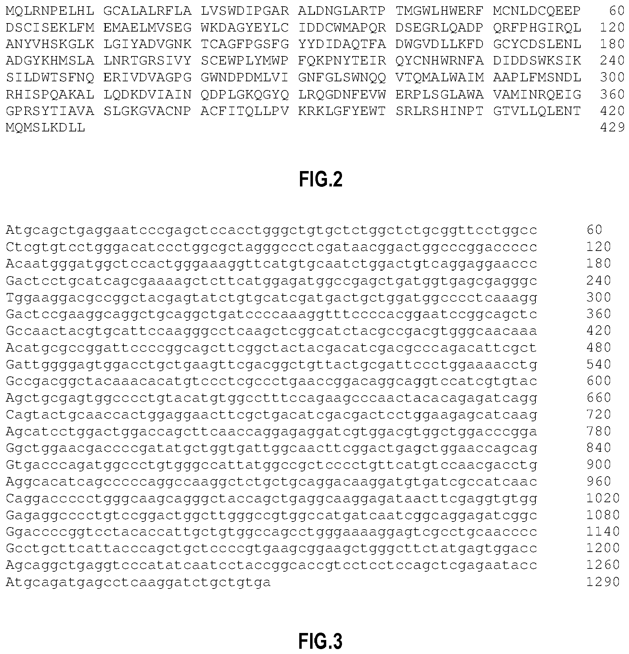

Human α-galactosidase A (α-Gal A) refers to an enzyme encoded by the human GLA gene. The full DNA sequence of α-Gal A, including introns and exons, is available in GenBank Accession No. X14448.1 and shown in FIG. 1A-E (SEQ ID NO: 1). The human α-Gal A enzyme consists of 429 amino acids and is available in GenBank Accession Nos. X14448.1 and U78027.1 and shown in FIG.2 (SEQ ID NO: 2). The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum (ER). The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the ER. The failure to achieve a stable conformation results in a substantial amount of the enzyme being degraded, rather than being transported to the lysosome.

As used herein, the term "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the ER to another cellular location, preferably a native cellular location, *i.e.,* prevents ER-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.,* chemical chaperones. In accordance with the present invention, the PC is migalastat or a salt thereof. In one embodiment, the PC is migalastat free base (*e.g.,* 123 mg of migalastat free base). In yet another embodiment, the PC is a salt of migalastat *(e.g.,* 150 mg of migalastat HCl).

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.,* competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.,* α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, *e.g*., co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.,* at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "α-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder (LSD), such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-GB3 and those disclosed in US Patent Application Publication No. U.S. 2010/0113517.

Non-limiting examples of improvements in surrogate markers for Fabry disease disclosed in U.S. 2010/0113517 include increases in α-Gal A levels or activity in cells (e.g., fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb₃; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; improvements in renal function; mitigation of hypohidrosis; the absence of angiokeratomas; and improvements in hearing abnormalities such as high frequency sensorineural hearing loss progressive hearing loss, sudden deafness, or tinnitus..

The dose that achieves one or more of the aforementioned responses is a "therapeutically effective dose."

The phrase "pharmaceutically acceptable" refers to molecular entities and compositions that are physiologically tolerable and do not typically produce untoward reactions when administered to a human. In some embodiments, as used herein, the term "pharmaceutically acceptable" means approved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

As used herein, the term "isolated" means that the referenced material is removed from the environment in which it is normally found. Thus, an isolated biological material can be free of cellular components, *i.e.,* components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, *e.g.,* suffering from enzyme insufficiency. The introduced enzyme may be a purified, recombinant enzyme produced *in vitro,* or protein purified from isolated tissue or fluid, such as, *e.g.,* placenta or animal milk, or from plants.

The term "ERT-naive patient" refers to a Fabry patient that has never received ERT or has not received ERT for at least 6 months prior to initiating migalastat therapy.

The term "ERT-experienced patient" refers to a Fabry patient that was receiving ERT immediately prior to initiating migalastat therapy. In some embodiments, the ERT-experienced patient has received at least 12 months of ERT immediately prior to initiating migalastat therapy.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts are expected to have different conversion factors, depending on the molecular weight of the salt.

The term "migalastat" encompasses migalastat free base or a pharmaceutically acceptable salt thereof (*e.g.*, migalastat HCl), unless specifically indicated to the contrary.

The terms "mutation" and "variant" (*e.g.*, as in "amenable mutation or variant") refer to a change in the nucleotide sequence of a gene or a chromosome. The two terms referred herein are typically used together - *e.g.,* as in "mutation or variant"- referring to the change in nucleotide sequence stated in the previous sentence. If only one of the two terms is recited for some reason, the missing term was intended to be included and one should understand as such. Furthermore, the terms "amenable mutation" and "amenable variant" refer to a mutation or variant that is amenable to PC therapy, *e.g.,* a mutation that is amenable to migalastat therapy. A particular type of amenable mutation or variant is a "HEK assay amenable mutation or variant", which is a mutation or variant that is determined to be amenable to migalastat therapy according to the criteria in the *in vitro* HEK assay described herein and in U.S. Patent No. 8,592,362.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked LSD. Mutations in the GLA gene result in a deficiency of the lysosomal enzyme, α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1,000 Fabry disease-causing GLA mutations have been identified. Approximately 60% are missense mutations, resulting in single amino acid substitutions in the α-Gal A enzyme. Missense GLA mutations often result in the production of abnormally folded and unstable forms of α-Gal A and the majority are associated with the classic phenotype. Normal cellular quality control mechanisms in the ER block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. The majority of currently treated patients are referred to as classic Fabry patients, most of whom are males. These patients experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as later-onset Fabry disease, tend to have higher residual α-Gal A levels than classic Fabry patients. Individuals with later-onset Fabry disease typically first experience disease symptoms in adulthood, and often have disease symptoms focused on a single organ, such as enlargement of the left ventricle or progressive kidney failure. In addition, later-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

In one or more embodiments, mutant forms of α-Gal A are considered to be amenable to migalastat are defined as showing a relative increase (+10 µM migalastat) of ≥1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (*e.g.*, migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (*e.g.*, migalastat) using these methods are listed in U.S. Patent No. 8,592,362.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582). In particular, administration of small molecule derivatives of glucose and galactose, which are specific, selective competitive inhibitors for several target lysosomal enzymes, effectively increased the stability of the enzymes in cells *in vitro* and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the ER (Ishii et al., Biochem. Biophys. Res. Comm. 1996; 220: 812-815), the enzyme protein is retarded in the normal transport pathway (ER→Golgi apparatus→endosomes→lysosome) and prematurely degraded. Therefore, a compound which binds to and increases the stability of a mutant enzyme, may serve as a "chaperone" for the enzyme and increase the amount that can exit the ER and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the ER and be trafficked to lysosomes.

The pharmacological chaperone in accordance with the present invention comprises migalastat or a salt thereof. The compound migalastat, also known as 1-deoxygalactonojirimycin (1-DGJ) or (2R,3S,4R,5S)-2-(hydroxymethyl) piperdine-3,4,5-triol is a compound having the following chemical formula:

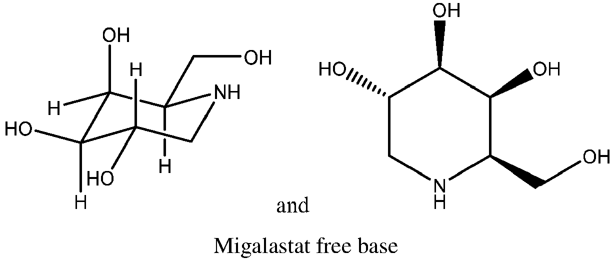

As discussed herein, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

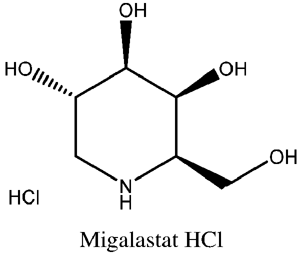

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 30-50% of patients with Fabry disease have HEK assay amenable mutations; the majority of which are associated with the classic phenotype of the disease.

HEK assay amenable mutations include at least those mutations listed in a pharmacological reference table *(e.g.,* the ones recited in the U.S. or International Product labels for a migalastat product such as GALAFOLD^{®}). As used herein, "pharmacological reference table" refers to any publicly accessible written or electronic record, included in either the product label within the packaging of a migalastat product (*e.g.*, GALAFOLD^{®}) or in a website accessible by health care providers, that conveys whether a particular mutation or variant is responsive to migalastat (*e.g*., GALAFOLD^{®}) PC therapy, and is not necessarily limited to written records presented in tabular form. In one embodiment of the present invention, a "pharmacological reference table" thus refers to any depository of information that includes one or more amenable mutations or variants. An exemplary pharmacological reference table for HEK assay amenable mutations can be found in the summary of product characteristics and/or prescribing information for GALAFOLD^{®} in various countries in which GALAFOLD^{®} is approved for use, or at a website such as www.galafoldamenabilitytable.com or www.fabrygenevariantsearch.com.

An exemplary pharmacological reference table for HEK assay amenable mutations is provided in Table 1 below. In one or more embodiments, if a double mutation is present on the same chromosome (males and females), that patient is considered HEK assay amenable if the double mutation is present in one entry in Table 1 (*e.g.*, D55V/Q57L). In some embodiments, if a double mutation is present on different chromosomes (only in females) that patient is considered HEK assay amenable if either one of the individual mutations is present in Table 1.

**Table 1**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.37G>A | c.G37A | A13T |
| c.37G>C | c.G37C | A13P |
| c.43G>A | c.G43A | A15T |
| c.44C>G | c.C44G | A15G |
| c.53T>G | c.T53G | F18C |
| c.58G>C | c.G58C | A20P |
| c.59C>A | c.C59A | A20D |
| c.70T>C or c.70T>A | c.T70C or c.T70A | W24R |
| c.70T>G | c.T70G | W24G |
| c.72G>C or c.72G>T | c.G72C or c.G72T | W24C |
| c.95T>C | c.T95C | L32P |
| c.97G>C | c.G97C | D33H |
| c.97G>T | c.G97T | D33Y |
| c.98A>G | c.A98G | D33G |
| c.100A>G | c.A100G | N34D |
| c.101A>C | c.A101C | N34T |
| c.101A>G | c.A101G | N34S |
| c.102T>G or c.102T>A | c.T102G or c.T102A | N34K |
| c.103G>C or c.103G>A | c.G103C or c.G103A | G35R |
| c.104G>A | c.G 104A | G35E |
| c.104G>C | c.G104C | G35A |
| c.104G>T | c.G104T | G35V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.110C>T | c.C110T | A37V |
| c.122C>T | c.C122T | T41I |
| c.124A>C or c.124A>T | c.A124C or c.A124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.137A>C | c.A137C | H46P |
| c.142G>C | c.G142C | E48Q |
| c.152T>A | c.T152A | M51K |
| c.153G>A or c.153G>T or c.153G>C | c.G153A or c.G153T or c.G153C | M51I |
| c.157A>G | c.A157G | N53D |
| c.[157A>C; 158A>T] | c.A157C/A158T | N53L |
| c.160C>T | c.C160T | L54F |
| c.161T>C | c.T161C | L54P |
| c.164A>G | c.A164G | D55G |
| c.164A>T | c.A164T | D55V |
| c.[164A>T; 170A>T] | c.A164T/A170T | D55V/Q57L |
| c.167G>T | c.G167T | C56F |
| c.167G>A | c.G167A | C56Y |
| c.170A>T | c.A170T | Q57L |
| c.175G>A | c.G175A | E59K |
| c.178C>A | c.C178A | P60T |
| c.178C>T | c.C178T | P60S |
| c.179C>T | c.C179T | P60L |
| c.196G>A | c.G196A | E66K |
| c.197A>G | c.A197G | E66G |
| c.207C>A or c.207C>G | c.C207A or c.C207G | F69L |
| c.214A>G | c.A214G | M72V |
| c.216G>A or c.216G>T or c.216G>C | c.G216A or c.G216T or c.G216C | M72I |
| c.218C>T | c.C218T | A73V |
| c.227T>C | c.T227C | M76T |
| c.239G>A | c.G239A | G80D |
| c.247G>A | c.G247A | D83N |
| c.253G>A | c.G253A | G85 S |
| c.254G>A | c.G254A | G85D |
| c.[253G>A; 254G>A] | c.G253A/G254A | G85N |
| c.[253G>A; 254G>T; 255T>G] | c.G253A/G254T/T255G | G85M |
| c.261G>C or c.261G>T | c.G261C or c.G261T | E87D |
| c.263A>C | c.A263C | Y88S |
| c.265C>T | c.C265T | L89F |
| c.272T>C | c.T272C | I91T |
| c.288G>A or c.288G>T or c.288G>C | c.G288A or c.G288T or c.G288C | M96I |
| c.289G>C | c.G289C | A97P |
| c.290C>T | c.C290T | A97V |
| c.305C>T | c.C305T | S 102L |
| c.311G>T | c.G311T | G104V |
| c.316C>T | c.C316T | L106F |
| c.322G>A | c.G322A | A108T |
| c.326A>G | c.A326G | D109G |
| c.334C>G | c.C334G | R112G |
| c.335G>A | c.G335A | R112H |
| c.337T>A | c.T337A | F113I |
| c.337T>C or c.339T>A or c.339T>G | c.T337C or c.T339A or c.T339G | F113L |
| c.352C>T | c.C352T | R118C |
| c.361G>A | c.G361A | A121T |
| c.368A>G | c.A368G | Y123C |
| c.373C>T | c.C373T | H125Y |
| c.374A>T | c.A374T | H125L |
| c.376A>G | c.A376G | S126G |
| c.383G>A | c.G383A | G128E |
| c.399T>G | c.T399G | I133M |
| c.404C>T | c.C404T | A135V |
| c.408T>A or c.408T>G | c.T408A or c.T408G | D136E |
| c.416A>G | c.A416G | N139S |
| c.419A>C | c.A419C | K140T |
| c.427G>A | c.G427A | A143T |
| c.431G>A | c.G431A | G144D |
| c.431G>T | c.G431T | G144V |
| c.434T>C | c.T434C | F145S |
| c.436C>T | c.C436T | P146S |
| c.437C>G | c.C437G | P146R |
| c.454T>C | c.T454C | Y152H |
| c.455A>G | c.A455G | Y152C |
| c.466G>A | c.G466A | A156T |
| c.467C>T | c.C467T | A156V |
| c.471G>C or c.471G>T | c.G471C or c.G471T | Q157H |
| c.484T>G | c.T484G | W162G |
| c.493G>C | c.G493C | D165H |
| c.494A>G | c.A494G | D165G |
| c.[496C>G; 497T>G] | c.C496G/T497G | L166G |
| c.496C>G | c.C496G | L166V |
| c.496_497delinsTC | c.496_497delinsTC | L166S |
| c.499C>G | c.C499G | L167V |
| c.506T>C | c.T506C | F169S |
| c.511G>A | c.G511A | G171S |
| c.520T>C | c.T520C | C174R |
| c.520T>G | c.T520G | C174G |
| c.525C>G or c.525C>A | c.C525G or c.C525A | D175E |
| c.539T>G | c.T539G | L180W |
| c.540G>C | c.G540C | L180F |
| c.548G>C | c.G548C | G183A |
| c.548G>A | c.G548A | G183D |
| c.550T>A | c.T550A | Y184N |
| c.551A>G | c.A551G | Y 184C |
| c.553A>G | c.A553G | K185E |
| c.559A>G | c.A559G | M187V |
| c.559_564dup | c.559_564dup | p.M187_S188dup |
| c.560T>C | c.T560C | M187T |
| c.561G>T or c.561G>A or c.561G>C | c.G561T or c.G561A or c.G561C | M187I |
| c.572T>A | c.T572A | L191Q |
| c.580A>G | c.A580G | T194A |
| c.581C>T | c.C581T | T194I |
| c.584G>T | c.G584T | G195V |
| c.586A>G | c.A586G | R196G |
| c.593T>C | c.T593C | I198T |
| c.595G>A | c.G595A | V199M |
| c.596T>C | c.T596C | V199A |
| c.596T>G | c.T596G | V199G |
| c.599A>G | c.A599G | Y200C |
| c.602C>T | c.C602T | S201F |
| c.602C>A | c.C602A | S201Y |
| c.608A>T | c.A608T | E203 V |
| c.609G>C or c.609G>T | c.G609C or c.G609T | E203D |
| c.610T>G | c.T610G | W204G |
| c.613C>A | c.C613A | P205T |
| c.613C>T | c.C613T | P205S |
| c.614C>T | c.C614T | P205L |
| c.619T>C | c.T619C | Y207H |
| c.620A>C | c.A620C | Y207S |
| c.623T>G | c.T623G | M208R |
| c.628C>T | c.C628T | P210S |
| c.629C>T | c.C629T | P210L |
| c.638A>G | c.A638G | K213R |
| c.638A>T | c.A638T | K213M |
| c.640C>T | c.C640T | P214S |
| c.641C>T | c.C641T | P214L |
| c.643A>G | c.A643G | N215D |
| c.644A>G | c.A644G | N215S |
| c.644A>T | c.A644T | N215I |
| c [644A>G; 937G>T] | c.A644G/G937T | N215S/D313Y |
| c.646T>G | c.T646G | Y216D |
| c.647A>C | c.A647C | Y216S |
| c.647A>G | c.A647G | Y216C |
| c.655A>C | c.A655C | I219L |
| c.656T>A | c.T656A | I219N |
| c.656T>C | c.T656C | I219T |
| c.659G>A | c.G659A | R220Q |
| c.659G>C | c.G659C | R220P |
| c.662A>C | c.A662C | Q221P |
| c.671A>C | c.A671C | N224T |
| c.671A>G | c.A671G | N224S |
| c.673C>G | c.C673G | H225D |
| c.683A>G | c.A683G | N228S |
| c.687T>A or c.687T>G | c.T687A or c.T687G | F229L |
| c.695 T>C | c.T695C | I232T |
| c.713G>A | c.G713A | S238N |
| c.716T>C | c.T716C | I239T |
| c.720G>C or c.720G>T | c.G720C or c.G720T | K240N |
| c.724A>G | c.A724G | I242V |
| c.724A>T | c.A724T | I242F |
| c.725T>A | c.T725A | I242N |
| c.725T>C | c.T725C | I242T |
| c.728T>G | c.T728G | L243 W |
| c.729G>C or c.729G>T | c.G729C or c.G729T | L243F |
| c.730G>A | c.G730A | D244N |
| c.730G>C | c.G730C | D244H |
| c.733T>G | c.T733G | W245G |
| c.740C>G | c.C740G | S247C |
| c.747C>G or c.747C>A | c.C747G or c.C747A | N249K |
| c.748C>A | c.C748A | Q250K |
| c.749A>C | c.A749C | Q250P |
| c.749A>G | c.A749G | Q250R |
| c.750G>C | c.G750C | Q250H |
| c.758T>C | c.T758C | I253T |
| c.758T>G | c.T758G | I253S |
| c.760-762delGTT | c.760_762delGTT | p.V254del |
| c.769G>C | c.G769C | A257P |
| c.770C>G | c.C770G | A257G |
| c.772G>C or c.772G>A | c.G772C or c.G772A | G258R |
| c.773G>T | c.G773T | G258V |
| c.776C>G | c.C776G | P259R |
| c.776C>T | c.C776T | P259L |
| c.779G>A | c.G779A | G260E |
| c.779G>C | c.G779C | G260A |
| c.781G>A | c.G781A | G261S |
| c.781G>C | c.G781C | G261R |
| c.781G>T | c.G781T | G261C |
| c.788A>G | c.A788G | N263S |
| c.790G>T | c.G790T | D264Y |
| c.794C>T | c.C794T | P265L |
| c.800T>C | c.T800C | M267T |
| c.805G>A | c.G805A | V269M |
| c.806T>C | c.T806C | V269A |
| c.809T>C | c.T809C | I270T |
| c.810T>G | c.T810G | I270M |
| c.811G>A | c.G811A | G271S |
| c.[811G>A; 937G>T] | c.G811A/G937T | G271S/D313Y |
| c.812G>A | c.G812A | G271D |
| c.823C>G | c.C823G | L275V |
| c.827G>A | c.G827A | S276N |
| c.829T>G | c.T829G | W277G |
| c.831G>T or c.831G>C | c.G831T or c.G831C | W277C |
| c.832A>T | c.A832T | N278Y |
| c.835C>G | c.C835G | Q279E |
| c.838C>A | c.C838A | Q280K |
| c.840A>T or c.840A>C | c.A840T or c.A840C | Q280H |
| c.844A>G | c.A844G | T282A |
| c.845C>T | c.C845T | T282I |
| c.850A>G | c.A850G | M284V |
| c.851T>C | c.T851C | M284T |
| c.860G>T | c.G860T | W287L |
| c.862G>C | c.G862C | A288P |
| c.866T>G | c.T866G | I289S |
| c.868A>C or c.868A>T | c.A868C or c.A868T | M290L |
| c.869T>C | c.T869C | M290T |
| c.870G>A or c.870G>C or c.870G>T | c.G870A or c.G870C or c.G870T | M290I |
| c.871G>A | c.G871A | A291T |
| c.877C>A | c.C877A | P293T |
| c.881T>C | c.T881C | L294S |
| c.884T>G | c.T884G | F295C |
| c.886A>G | c.A886G | M296V |
| c.886A>T or c.886A>C | c.A886T or c.A886C | M296L |
| c.887T>C | c.T887C | M296T |
| c.888G>A or c.888G>T or c.888G>C | c.G888A or c.G888T or c.G888C | M296I |
| c.893A>G | c.A893G | N298S |
| c.897C>G or c.897C>A | c.C897G or c.C897A | D299E |
| c.898C>T | c.C898T | L300F |
| c.899T>C | c.T899C | L300P |
| c.901C>G | c.C901G | R301G |
| c.902G>C | c.G902C | R301P |
| c.902G>A | c.G902A | R301Q |
| c.902G>T | c.G902T | R301L |
| c.907A>T | c.A907T | I303F |
| c.908T>A | c.T908A | I303N |
| c.911G>A | c.G911A | S304N |
| c.911G>C | c.G911C | S304T |
| c.919G>A | c.G919A | A307T |
| c.922A>G | c.A922G | K308E |
| c.924A>T or c.924A>C | c.A924T or c.A924C | K308N |
| c.925G>C | c.G925C | A309P |
| c.926C>T | c.C926T | A309V |
| c.928C>T | c.C928T | L310F |
| c.931C>G | c.C931G | L311V |
| c.935A>G | c.A935G | Q312R |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | c.A938G | D313G |
| c.946G>A | c.G946A | V316I |
| c.947T>G | c.T947G | V316G |
| c.950T>C | c.T950C | I317T |
| c.955A>T | c.A955T | I319F |
| c.956T>C | c.T956C | I319T |
| c.959A>T | c.A959T | N320I |
| c.962A>G | c.A962G | Q321R |
| c.962A>T | c.A962T | Q321L |
| c.963G>C or c.963G>T | c.G963C or c.G963T | Q321H |
| c.964G>A | c.G964A | D322N |
| c.964G>C | c.G964C | D322H |
| c.966C>A or c.966C>G | c.C966A or c.C966G | D322E |
| c.968C>G | c.C968G | P323R |
| c.973G>A | c.G973A | G325S |
| c.973G>C | c.G973C | G325R |
| c.978G>C or c.978G>T | c.G978C or c.G978T | K326N |
| c.979C>G | c.C979G | Q327E |
| c.980A>T | c.A980T | Q327L |
| c.983G>C | c.G983C | G328A |
| c.989A>G | c.A989G | Q330R |
| c.1001G>A | c.G1001A | G334E |
| c.1010T>C | c.T1010C | F337S |
| c.1012G>A | c.G1012A | E338K |
| c.1016T>A | c.T1016A | V339E |
| c.1027C>A | c.C1027A | P343T |
| c.1028C>T | c.C1028T | P343L |
| c.1033T>C | c.T1033C | S345P |
| c.1046G>C | c.G1046C | W349S |
| c.1055C>G | c.C1055G | A352G |
| c.1055C>T | c.C1055T | A352V |
| c.1061T>A | c.T1061A | I354K |
| c.1066C>G | c.C1066G | R356G |
| c.1066C>T | c.C1066T | R356W |
| c.1067G>A | c.G1067A | R356Q |
| c.1067G>C | c.G1067C | R356P |
| c.1072G>C | c.G1072C | E358Q |
| c.1073A>C | c.A1073C | E358A |
| c.1073A>G | c.A1073G | E358G |
| c.1074G>T or c.1074G>C | c.G1074T or c.G1074C | E358D |
| c.1076T>C | c.T1076C | I359T |
| c.1078G>A | c.G1078A | G360S |
| c.1078G>T | c.G1078T | G360C |
| c.1079G>A | c.G1079A | G360D |
| c.1082G>A | c.G1082A | G361E |
| c.1082G>C | c.G1082C | G361A |
| c.1084C>A | c.C1084A | P362T |
| c.1085C>T | c.C108ST | P362L |
| c.1087C>T | c.C1087T | R363C |
| c.1088G>A | c.G1088A | R363H |
| c.1102G>A | c.G1102A | A368T |
| c.1117G>A | c.G1117A | G373S |
| c.1124G>A | c.G1124A | G375E |
| c.1153A>G | c.A1153G | T385A |
| c.1168G>A | c.G1168A | V390M |
| c.1172A>C | c.A1172C | K391T |
| c.1175G>C | c.G1175C | R392T |
| c.1184G>A | c.G1184A | G395E |
| c.1184G>C | c.G1184C | G395A |
| c.1192G>A | c.G1192A | E398K |
| c.1202_1203insGACTTC | c.1202_1203insGACTTC | p.T400_S401dup |
| c.1208T>C | c.T1208C | L403S |
| c.1225C>G | c.C1225G | P409A |
| c.1225C>T | c.C1225T | P409S |
| c.1225C>A | c.C1225A | P409T |
| c.1228A>G | c.A1228G | T410A |
| c.1229C>T | c.C1229T | T410I |
| c.1232G>A | c.G1232A | G411D |
| c.1235C>A | c.C1235A | T412N |
| c.1253A>G | c.A1253G | E418G |
| c.1261A>G | c.A1261G | M421V |

### Dosing, Formulation and Administration

In accordance with the present invention, the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt. In some embodiments, these doses pertain to the free base of migalastat. In alternate embodiments, these doses pertain to a salt of migalastat. In further embodiments, the salt of migalastat is migalastat hydrochloride. The administration of migalastat or a salt of migalastat is referred to herein as "migalastat therapy".

In accordance with the present invention, the effective amount of migalastat or salt thereof is in the range from about 100 mg FBE to about 150 mg FBE. Exemplary doses include about 100 mg FBE, about 105 mg FBE, about 110 mg FBE, about 115 mg FBE, about 120 mg FBE, about 123 mg FBE, about 125 mg FBE, about 130 mg FBE, about 135 mg FBE, about 140 mg FBE, about 145 mg FBE or about 150 mg FBE.

Again, it is noted that 150 mg of migalastat hydrochloride is equivalent to 123 mg of the free base form of migalastat. Thus, in one or more embodiments, the dose is 150 mg of migalastat hydrochloride or an equivalent dose of migalastat or a salt thereof other than the hydrochloride salt, administered at a frequency of once every other day. As set forth above, this dose is referred to as 123 mg FBE of migalastat. In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day.

The administration of migalastat or salt thereof may be for a certain period of time. In one or more embodiments, the migalastat or salt thereof is administered for a duration of at least 24, 30 or 36 months or at least 2, 3, 4 or 5 years. In various embodiments, the migalastat therapy is a long-term migalastat therapy of at least about 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, the PC (migalastat or salt thereof) is administered orally. In one or more embodiments, the PC (migalastat or salt thereof) is administered by injection. The PC may be accompanied by a pharmaceutically acceptable carrier, which may depend on the method of administration.

In one or more embodiments, the PC (migalastat or salt thereof) is administered as monotherapy, and can be in a form suitable for any route of administration, including *e.g.,* orally in the form tablets or capsules or liquid, or in sterile aqueous solution for injection. In other embodiments, the PC is provided in a dry lyophilized powder to be added to the formulation of the replacement enzyme during or immediately after reconstitution to prevent enzyme aggregation *in vitro* prior to administration.

When the PC (migalastat or salt thereof) is formulated for oral administration, the tablets or capsules can be prepared by conventional means with pharmaceutically acceptable excipients such as binding agents (*e.g.*, pregelatinized maize starch, polyvinylpyrrolidone or hydroxypropyl methylcellulose); fillers *(e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.,* sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC (migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC (migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, bicarbonate buffer, amino acids, urea, alcohols, ascorbic acid, and phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol *(e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external *(e.g.,* an i.v. bag) or internal *(e.g.,* a bioerodable implant).

Embodiments relating to pharmaceutical formulations and administration may be combined with any of the other embodiments of the invention, for example embodiments relating to methods of treating patients with Fabry disease, methods of treating ERT-naive Fabry patients, methods of treating ERT-experienced Fabry patients, methods of reducing the risk of CBV events, methods of treating Fabry patients with increased risk of CBV events, methods of treating Fabry patients with a history of CBV events, methods of treating Fabry patients without a history of CBV events, methods of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC (migalastat or salt thereof) is administered in combination with ERT. ERT increases the amount of protein by exogenously introducing wild-type or biologically functional enzyme by way of infusion. This therapy has been developed for many genetic disorders, including LSDs such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g.,* replacement α-Gal A). In some embodiments, the chaperone is co-formulated with the replacement enzyme (*e.g.,* replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein.

### CBV Events

The dosing regimens described herein can reduce the risk of CBV events in Fabry patients. CBV events, including but not limited to stroke and transient ischemic attack, are serious clinical events common among patients with Fabry disease, occurring in -18% of untreated patients over a mean follow-up of 4.5 years as per a meta-analysis by El Dib, et al (El Dib R. PLoS One. 2017;12(3):e0173358). Incidence of CBV events in patients receiving ERT has been reported to range from 2% to 27 %. However, the effect of ERT on the risk of CBV events remains unknown due to the paucity of placebo-controlled studies, and recombinant lysosomal enzyme has not been shown to cross the blood-brain barrier.

As described in further detail in the Examples below, Phase 2 and 3 studies have found that migalastat therapy reduces the incidence of CBV events in both ERT-experienced and ERT-naive patients. Accordingly, migalastat therapy can be used to reduce the risk of CBV events and/or treat Fabry patients that have a high risk of CBV events, including for patients that have a history of CBV events or patients that do not have a history of CBV events.

### EXAMPLES

### EXAMPLE 1: Dosing Regimens for the Treatment of ERT-Experienced and ERT-Naive Fabry Patients Using Migalastat Hydrochloride

This example describes Phase 2 and Phase 3 studies of migalastat therapy in ERT-experienced and ERT-naive Fabry patients.

### Study Designs

These analyses included data from 4 Phase 2 and 4 Phase 3 clinical trials with the data cutoff of February 10, 2017 as shown in Figure X1 below.

FAB-CL-202 (NCT00283959), FAB-CL-203 (NCT00283933), and FAB-CL-204 (NCT00304512) were phase 2, open-label, noncomparative studies that evaluated the safety, tolerability, pharmacokinetics (PK), and pharmacodynamics (PD) of migalastat (dose range: 50-250 mg) in patients with Fabry disease.

FAB-CL-205 (NCT0052607) was a phase 2, long-term, open-label extension (OLE) study for patients completing phase 2 clinical trials, including FAB-CL-202, FAB-CL-203, and FAB-CL-204. The study included a period with migalastat 150 mg every other day (QOD), then a dose-escalation period, followed by 150 mg QOD.

FACETS (AT1001-011, NCT00925301) was a phase 3, placebo-controlled study designed to evaluate the efficacy, safety, and PD of 6 months of migalastat 150 mg QOD versus placebo, followed by an 18-month open label extension (OLE) of migalastat in ERT-naive patients with Fabry disease and migalastat-amenable GLA variants.

ATTRACT (AT1001-012, NCT01218659) was a phase 3, open-label, active-controlled study to compare the efficacy and safety of 18 months of migalastat 150 mg QOD versus ERT, followed by a 12-month OLE of migalastat, in ERT-treated patients with migalastat-amenable GLA variants.

AT1001-041 (NCT01458119) was a long-term OLE study evaluating the long-term safety and efficacy of migalastat in patients completing FAB-CL-205, AT1001-011, or AT1001-012

AT1001-042 (NCT02194985) is an ongoing, long-term OLE study evaluating the long-term safety and efficacy of migalastat in patients who participated in AT1001-012 or AT1001-041.

### Analyses

The analysis evaluates CBV events reported as treatment-emergent adverse events (TEAEs) during migalastat 150 mg QOD treatment in patients with amenable mutations in phase 2 and phase 3 clinical trials.

CBV events were identified by searching medical history and TEAE listings with stroke-related terms, including brain stem ischemia, cerebral infarction, cerebral hemorrhage, cerebral ischemia, cerebrovascular accident, embolic stroke, and TIA.

Only amenable patients who received at least 1 dose of migalastat 150 mg QOD were included in this analysis.

Amenability was based on results from a good laboratory practice (GLP)-validated, in vitro migalastat amenability assay.

### Clinical Studies Included in the Analysis

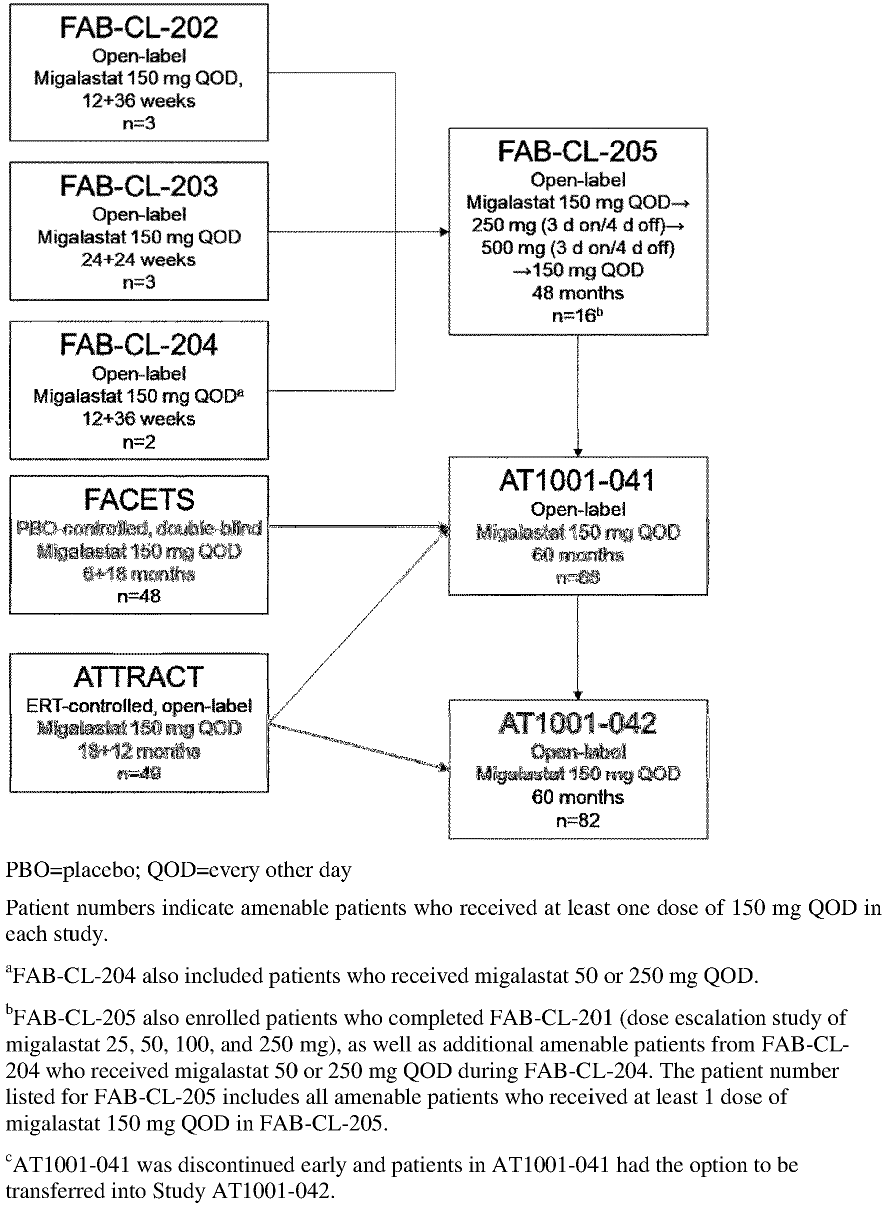
FAB-CL-202
Open-label
Migalastat 150 mg QOD,
12+36 weeks
n=3

FAB-CL-203
Open-label
Migalastat 150 mg QOD
24+24 weeks
n=3

FAB-CL-204
Open-label
Migalastat 150 mg QOD^a
12+36 weeks
n=2

FACETS
PBO-controlled, double-blind
Migalastat 150 mg QOD
6+18 months
n=48

ATTRACT
ERT-controlled, open-label
Migalastat 150 mg QOD
18+12 months
n=49

FAB-CL-205
Open-label
Migalastat 150 mg QOD→
250 mg (3 d on/4 d off)→
500 mg (3 d on/4 d off)
→150 mg QOD
48 months
n=16^b

AT1001-041
Open-label
Migalastat 150 mg QOD
60 months
n=68

AT1001-042
Open-label
Migalastat 150 mg QOD
60 months
n=82


PBO=placebo; QOD=every other day

Patient numbers indicate amenable patients who received at least one dose of 150 mg QOD in
each study.

^aFAB-CL-204 also included patients who received migalastat 50 or 250 mg QOD.

^bFAB-CL-205 also enrolled patients who completed FAB-CL-201 (dose escalation study of
migalastat 25, 50, 100, and 250 mg), as well as additional amenable patients from FAB-CL-
204 who received migalastat 50 or 250 mg QOD during FAB-CL-204. The patient number
listed for FAB-CL-205 includes all amenable patients who received at least 1 dose of
migalastat 150 mg QOD in FAB-CL-205.

^cAT1001-041 was discontinued early and patients in AT1001-041 had the option to be
transferred into Study AT1001-042.

FAB-CL-202, FAB-CL-203, FAB-CL-204 and FAB-CL-205 are Phase 2 clinical studies; FACETS, ATTRACT, AT1001-41 AND AT1001-042 are Phase 3 clinical studies

### Results

### Migalastat 150 mg QOD Total Exposure

The total mean (SD) duration of exposure to migalastat 150 mg QOD was 4.0 (2.0) years (N=114).

The duration of exposure to migalastat 150 mg QOD ranged from 0.1 to 8.3 years, with a median of 4.4 years.

### Demographics and Baseline Characteristics

The mean (SD) age of all amenable patients receiving at least 1 dose of migalastat 150 mg QOD was 46.2 (13.1) years (range: 16 to 72 years) (Table 2). The majority were white, and 57.0% were female. The mean (SD) time since diagnosis of Fabry disease was 9.8 (10.1) years (range: 1 to 44 years).

**Table 2. Demographics and Baseline Characteristics: All Amenable Patients Receiving Migalastat 150 mg QOD**

| **Parameter** | | **FAB-CL 202, 203, 204, and 205 (n=17)** | **FACETS (n=48)** | **ATTRACT (n=49)** | **Total (N=114)** |
|---|---|---|---|---|---|
| **Age, year** | | | | | |
| | Mean (SD) | 43.1 (13.4) | 43.4 (11.2) | 50.0 (14.0) | 46.2 (13.1) |
| | Median (range) | 42.0 (18, 65) | 46.0 (16, 68) | 54.0 (18, 72) | 46.5 |

| **Age group, n (%)** | | | | | |
|---|---|---|---|---|---|
| | <18 years old | 0 | 1 (2.1) | 0 | 1 (0.9) |
| | 18 to <65 years old | 15 (88.2) | 46 (95.8) | 42 (85.7) | 103 (90.4) |
| | ≥65 years old | 2 (11.8) | 1 (2.1) | 7 (14.3) | 10(8.8) |

| **Sex, n** (**%**) | | | | | |
|---|---|---|---|---|---|
| | Female | 5 (29.4) | 30 (62.5) | 30 (61.2) | 65 (57.0) |
| | Male | 12 (70.6) | 18 (37.5) | 19 (38.8) | 49 (43.0) |

| **Years diagnosed with Fabry disease** | | | | | |
|---|---|---|---|---|---|
| | Mean (SD) | 7.2 (7.5) | 7.6 (7.8)^{a} | 12.9 (12.1) | 9.8 (10.1) |
| | Median (range) | 5.0 (2, 34) | 5.0 (1, 25) | 7.0 (3, 44) | 6.0 (1, 44) |

| | | | | | |
|---|---|---|---|---|---|
| ACEI=angiotensin-converting enzyme inhibitor; ARB=angiotensin receptor block; RI=renin inhibitor; SD=standard deviation. ^{a}Fabry disease diagnosis date was not recorded for 1 patient in FACETS. | | | | | |

### Medical History of CBV Events

Sixteen of 114 patients (14%) had experienced CBV events prior to migalastat treatment (Table 3). One patient from Study AT1001-012 had reported 2 CBV events in medical history.

In 5/16 patients, the CBV events were considered a current condition at study entry as reported in medical history. One patient from AT1001-011 had ongoing cerebral ischemia; another had ongoing brain stem infarction. Two patients from AT1001-012 had ongoing TIA, one had an ongoing cerebrovascular accident, specifically left middle cerebral artery stroke

The mean (SD) age at the time of first CBV event was 43.6 (14.4) years.

**Table 2. Medical History of CBV Events**

| | **FAB-CL 202, 203, 204, and 205 (n=17)** | **FACETS (n=48)** | **ATTRACT (n=49)** | **Total (N=114)** |
|---|---|---|---|---|
| Brain stem ischemia | 0 | 0 | 0 | 0 |
| Cerebral infarction | 0 | 1 (2%) | 0 | 1 (1%) |
| Cerebral hemorrhage | 0 | 0 | 0 | 0 |
| Cerebral ischemia | 0 | 1 (2%) | 1 (2%) | 2 (2%) |
| Cerebrovascular accident | 0 | 2 (4%) | 3 (6%) | 5 (4%) |
| Embolic stroke | 0 | 0 | 0 | 0 |
| Transient ischemic attack | 2 (12%) | 2 (4%) | 5 (10%) | 9 (8%) |
| **Any CBV event history^{a}** | **2 (12%)** | **6 (12%)** | **8 (16%)** | **16 (14%)** |

| | | | | |
|---|---|---|---|---|
| ^{a}The last row shows number of unique patients with CBV events. The 1 patient with >1 CBV event was only counted once. | | | | |

### Occurrence of CBV Events During Migalastat 150 mg QOD Treatment

Eleven CBV events were reported during treatment with migalastat 150 mg QOD in 8 patients (7%) (Table 4). Seven CBV events were categorized as serious adverse events (SAE); however, most (82%) events were mild or moderate in severity (Table 5). Two CBV events led to treatment discontinuation (Table 5). None of the 11 CBV events were considered related to treatment

Six out of the 8 patients had experienced CBV prior to receiving migalastat treatment; thus only 2/114 (2%) patients had a first CBV event while on migalastat (Table 5). The mean (SD) age of patients at first event during migalastat treatment was 50.6 (14.6) years (Table 5). The mean (SD) time on migalastat 150 mg QOD at first event onset was 1.1 (1.1) years.

Among the 16 patients with pre-migalastat CBV event, 10 (63%) did not experience new CBV event during migalastat treatment.

**Table 4. CBV Events During Treatment With Migalastat 150 mg QOD by Trial**

| | **FAB-CL 202, 203, 204, and 205 (n=17)** | **FACETS (n=48)** | **ATTRACT (n=49)** | **Total (N=114)** |
|---|---|---|---|---|
| Brain stem ischemia | 0 | 1 (2%) | 0 | 1 (1%) |
| Cerebral infarction | 1 (6%) | 0 | 0 | 1 (1%) |
| Cerebral hemorrhage | 0 | 1 (2%) | 0 | 1 (1%) |
| Cerebral ischemia | 1 (6%) | 0 | 0 | 1 (1%) |
| Cerebrovascular accident | 1 (6%) | 0 | 0 | 1 (1%) |
| Embolic stroke | 0 | 0 | 0 | 0 |
| Transient ischemic attack | 1 (6%) | 1 (2%) | 2 (4%) | 4 (4%) |
| **Any CBV event^{a}** | **3 (18 %)** | **3 (6%)** | **2 (4%)** | **8 (7%)** |

| | | | | |
|---|---|---|---|---|
| ^{a}The last row shows number of unique patients with CBV events. Patients with >1 CBV event were only counted once. | | | | |

**Table 5 CBV Events During Treatment With Migalastat 150 mg QOD by Patient**

| **Patient No.** | **Sex** | **Mutation** | **CBV Event** | **Event duration (day)** | **Age at event onset (year)** | **Time on migalast at 150 mg QOD at onset (day)** | **Serious** | **Severity** | **Resolved** | **Treatment discontinued** | **History of CBV event** |
|---|---|---|---|---|---|---|---|---|---|---|---|
| 1 | F | M284T | TIA (right eye) | 3 | 38 | 589 | Yes | Mild | Yes | No | CBV accident |
| 2 | M | I253T | Cerebral hemorrhage | Ongoing^{b} | 63 | 289 | Yes | Severe | No | Yes | None |
| 3 | M | I253T | Brain stem ischemia (left pontine region) | 47 | 66 | 1413 | Yes | Moderate | Yes with sequela | No | Cerebral ischemia |
| 4 | M | G35R | TIA | 7 | 59 | 24 | Yes | Moderate | Yes | No | TIA |
| 5 | F | L32P | TIA | 1 | 56 | 559 | No | Mild | Yes | No | TIA |
| | | | TIA | 1 | 56 | 624 | No | Moderate | Yes | No | |
| 6 | M | P259R | CBV accident | 220 | 21 | 90 | Yes | Severe | Yes with sequela | Yes | None |
| 7 | F | R112H | TIA | 3 | 39 | 21 | Yes | Moderate | Yes | No | TIA |
| | | | TIA | 2 | 41 | 666 | Yes | Moderate | Yes | No | |
| 8 | F | P205T | Cerebral infarction (right middle artery) | Ongoing^{c} | 60 | 371 | No | Moderate | No | No | TIA |
| | | | Abnormal MRI finding (old ischemic change of right frontal lobe) | Ongoing^{c} | 60 | 182 | No | Moderate | No | No | |

| **Patient No.** | **Baseline Parameters^{a}** | | | | | |
|---|---|---|---|---|---|---|
| | **Time since diagnosis (year)** | **Blood Pressure (mm Hg)** | **Urine Protein (mg/24 hr)** | **eGFR_{CKD-EPI} (mL/min/1.73 m²)** | **LVMi (g/m²)** | **ACEI/ARB/RI use** |
| 1 | 14.5 | 120/80 | 399 | 81.8 | 88.7 | No |
| 2 | 0.3 | NA | 1900 | 53.8 | 142.8 | No |
| 3 | 4.6 | 120/80 | 331 | 86.8 | 176.2 | Yes |
| 4 | 10 | 110/67 | 182 | 84.9 | 154.9 | Yes |
| 5 | 2.2 | 112/64 | 231 | 73.2 | 68.6 | Yes |
| 6 | 8.3 | 110/70 | 66 | 137.4 | NA | No |
| 7 | 5.8 | 113/73 | 163 | 120.0 | NA | No |
| 8 | 1.9 | 129/69 | NA | NA^{d} | NA | No |

| | | | | | | |
|---|---|---|---|---|---|---|
| LVMi=left ventricular mass index; MRI=magnetic resonance imaging; NA=not applicable; TIA=transient ischemic attack. ^{a}Baseline was the start of migalastat 150 mg QOD. ^{b}Ongoing at the time of discontinuation in FACETS. ^{c}Ongoing at the end of FAB-CL-204. ^{d}This patient had an eGFR_{MDRD} of 76.4 mL/min/1.73 m². | | | | | | |

As can be seen from the tables above, overall incidence of CBV events was low during migalastat treatment. During an average of 4 years of migalastat, 8/114 (7%) patients had experienced CBV events, predominantly occurring in patients with a history of CBV events.

The embodiments described herein are intended to be illustrative of the present compositions and methods and are not intended to limit the scope of the present invention. Various modifications and changes consistent with the description as a whole and which are readily apparent to the person of skill in the art are intended to be included. The appended claims should not be limited by the specific embodiments set forth in the examples, but should be given the broadest interpretation consistent with the description as a whole.

Patents, patent applications, publications, product descriptions, GenBank Accession Numbers, and protocols are cited throughout this application.

## Claims

1. Migalastat or salt thereof for use in a method of reducing the risk of a cerebrovascular (CBV) event in a patient having Fabry disease, the method comprising administering to the patient a formulation comprising an effective amount of the migalastat or salt thereof every other day for at least 2 years, wherein the effective amount is about 100 mg to about 150 mg free base equivalent (FBE).

2. Migalastat or salt thereof for use according to claim 1, wherein the CBV event comprises one or more of brain stem ischemia, cerebral infarction, cerebral hemorrhage, cerebral ischemia, cerebrovascular accident, embolic stroke or transient ischemic attack.

3. Migalastat or salt thereof for use according to claim 1 or 2, wherein the migalastat or salt thereof enhances α-galactosidase A activity.

4. Migalastat or salt thereof for use according to any one of claims 1-3, wherein the patient is administered about 123 mg FBE of the migalastat or salt thereof every other day.

5. Migalastat or salt thereof for use according to any one of claims 1-4, wherein the patient is administered about 150 mg of migalastat hydrochloride every other day.

6. Migalastat or salt thereof for use according to any one of claims 1-5, wherein the formulation comprises an oral dosage form.

7. Migalastat or salt thereof for use according to claim 6, wherein the oral dosage form comprises a tablet, a capsule or a solution.

8. Migalastat or salt thereof for use according to any one of claims 1-7, wherein the migalastat or salt thereof is administered for at least 3 years or for at least 4 years.

9. Migalastat or salt thereof for use according to any one of claims 1-8, wherein the patient did not have a first CBV event prior to initiating administration of the migalastat or salt thereof.

10. Migalastat or salt thereof for use according to any one of claims 1-8, wherein the patient had a first CBV event prior to initiating the administration of the migalastat or salt thereof.

11. Migalastat or salt thereof for use according to any one of claims 1-10, wherein the patient is an enzyme replacement therapy (ERT)-naïve patient.

12. Migalastat or salt thereof for use according to any one of claims 1-10, wherein the patient is an ERT-experienced patient.

13. Migalastat or salt thereof for use according to any one of claims 1-12, wherein the patient has a HEK assay amenable mutation in α-galactosidase A.

14. Migalastat or salt thereof for use according to claim 13, wherein the mutation is disclosed in a pharmacological reference table.

15. Migalastat or salt thereof for use according to claim 14, wherein the pharmacological reference table is provided in a product label for a migalastat product approved for the treatment of Fabry disease.

## Patentansprüche

1. Migalastat oder ein Salz davon zur Verwendung in einem Verfahren zur Verringerung des Risikos eines zerebrovaskulären (CBV) Events bei einem Patienten mit Morbus Fabry, wobei das Verfahren die Verabreichung einer Formulierung, die eine wirksame Menge des Migalastats oder eines Salzes davon umfasst, an den Patienten jeden zweiten Tag für mindestens 2 Jahre umfasst, wobei die wirksame Menge etwa 100 mg bis etwa 150 mg freies Basenäquivalent (FBE) ist.

2. Migalastat oder ein Salz davon zur Verwendung gemäß Anspruch 1, wobei das CBV-Event eines oder mehrere von Hirnstammischämie, Hirninfarkt, Hirnblutung, zerebraler Ischämie, zerebrovaskulärem Unfall, embolischem Schlaganfall oder transitorischer ischämischer Attacke umfasst.

3. Migalastat oder ein Salz davon zur Verwendung gemäß Anspruch 1 oder 2, wobei das Migalastat oder ein Salz davon die Aktivität von α-Galactosidase A verstärkt.

4. Migalastat oder ein Salz davon zur Verwendung gemäß irgendeinem der Ansprüche 1-3, wobei dem Patienten etwa 123 mg FBE des Migalastats oder eines Salzes davon jeden zweiten Tag verabreicht werden.

5. Migalastat oder ein Salz davon zur Verwendung gemäß irgendeinem der Ansprüche 1-4, wobei dem Patienten etwa 150 mg Migalastathydrochlorid jeden zweiten Tag verabreicht werden.

6. Migalastat oder ein Salz davon zur Verwendung gemäß irgendeinem der Ansprüche 1-5, wobei die Formulierung eine orale Dosierungsform umfasst.

7. Migalastat oder ein Salz davon zur Verwendung gemäß Anspruch 6, wobei die orale Dosierungsform eine Tablette, eine Kapsel oder eine Lösung umfasst.

8. Migalastat oder ein Salz davon zur Verwendung gemäß irgendeinem der Ansprüche 1-7, wobei das Migalastat oder ein Salz davon für mindestens 3 Jahre oder für mindestens 4 Jahre verabreicht wird.

9. Migalastat oder ein Salz davon zur Verwendung gemäß irgendeinem der Ansprüche 1-8, wobei der Patient vor Beginn der Verabreichung von Migalastat oder einem Salz davon kein erstes CBV-Event hatte.

10. Migalastat oder ein Salz davon zur Verwendung gemäß irgendeinem der Ansprüche 1-8, wobei der Patient vor Beginn der Verabreichung von Migalastat oder einem Salz davon ein erstes CBV-Event hatte.

11. Migalastat oder ein Salz davon zur Verwendung gemäß irgendeinem der Ansprüche 1-10, wobei der Patient ein Enzymersatztherapie (ERT) naiver Patient ist.

12. Migalastat oder ein Salz davon zur Verwendung gemäß irgendeinem der Ansprüche 1-10, wobei der Patient ein (ERT) erfahrener Patient ist.

13. Migalastat oder ein Salz davon zur Verwendung gemäß irgendeinem der Ansprüche 1-12, wobei der Patient in α-Galactosidase A eine Mutation aufweist, die für einen HEK-Assay zugänglich ist.

14. Migalastat oder ein Salz davon zur Verwendung gemäß Anspruch 13, wobei die Mutation in einer pharmakologischen Referenztabelle offenbart ist.

15. Migalastat oder ein Salz davon zur Verwendung gemäß Anspruch 14, wobei die pharmakologische Referenztabelle in einem Produktetikett für ein Migalastat-Produkt bereitgestellt wird, das für die Behandlung von Morbus Fabry zugelassen ist.

## Revendications

1. Migalastat ou sel de celui-ci à utiliser dans un procédé de réduction du risque d'un événement cérébrovasculaire (CBV) chez un patient ayant la maladie de Fabry, le procédé comprenant l'administration au patient d'une formulation comprenant une quantité efficace de migalastat ou de sel de celui-ci tous les deux jours pendant au moins 2 ans, dans lequel la quantité efficace est environ 100 mg à environ 150 mg d'équivalent de base libre (FBE).

2. Migalastat ou sel de celui-ci à utiliser selon la revendication 1, dans lequel l'événement CBV comprend un ou plusieurs parmi une ischémie du tronc cérébral, un infarctus cérébral, une hémorragie cérébrale, une ischémie cérébrale, un accident vasculaire cérébral (AVC), un AVC embolique ou un accident ischémique transitoire.

3. Migalastat ou sel de celui-ci à utiliser selon la revendication 1 ou la revendication 2, dans lequel le migalastat ou le sel de celui-ci améliore l'activité d'α-galactosidase A.

4. Migalastat ou sel de celui-ci à utiliser selon l'une des revendications 1 à 3, dans lequel il est administré au patient environ 123 mg FBE de migalastat ou de sel de celui-ci tous les deux jours.

5. Migalastat ou sel de celui-ci à utiliser selon l'une des revendications 1 à 4, dans lequel il est administré au patient environ 150 mg de chlorhydrate de migalastat tous les deux jours.

6. Migalastat ou sel de celui-ci à utiliser selon l'une des revendications 1 à 5, dans lequel la formulation comprend une forme galénique orale.

7. Migalastat ou sel de celui-ci à utiliser selon la revendication 6, dans lequel la forme galénique orale comprend un comprimé, une pilule ou une solution.

8. Migalastat ou sel de celui-ci à utiliser selon l'une des revendications 1 à 7, dans lequel le migalastat ou le sel de celui-ci est administré pendant au moins 3 ans ou pendant au moins 4 ans.

9. Migalastat ou sel de celui-ci à utiliser selon l'une des revendications 1 à 8, dans lequel le patient n'avait pas connu de premier événement CBV avant de commencer l'administration de migalastat ou de sel de celui-ci.

10. Migalastat ou sel de celui-ci à utiliser selon l'une des revendications 1 à 8, dans lequel le patient avait connu un premier événement CBV avant de commencer l'administration de migalastat ou de sel de celui-ci.

11. Migalastat ou sel de celui-ci à utiliser selon l'une des revendications 1 à 10, dans lequel le patient n'a jamais reçu d'enzymothérapie de substitution (ERT).

12. Migalastat ou sel de celui-ci à utiliser selon l'une des revendications 1 à 10, dans lequel le patient a déjà reçu une ERT.

13. Migalastat ou sel de celui-ci à utiliser selon l'une des revendications 1 à 12, dans lequel le patient présente une mutation dans α-galactosidase A réactive au test HEK.

14. Migalastat ou sel de celui-ci à utiliser selon la revendication 13, dans lequel la mutation est divulguée dans une table de références pharmacologiques.

15. Migalastat ou sel de celui-ci à utiliser selon la revendication 14, dans lequel la table de références pharmacologiques est fournie dans une étiquette de produit pour un produit migalastat approuvé pour le traitement de la maladie de Fabry.
